# EUROPEAN PATENT SPECIFICATION

(11) **EP 2 224 976 B1**
(45) Date of publication and mention of the grant of the patent: **20.02.2013**
(21) Application number: 08864670.8
(22) Date of filing: 19.12.2008
(51) Int. Cl.: A61M 3/02, A61M 25/10

(54) **DIAMETER-CONTROLLED BALLOON CATHETER**
BALLONKATHETER MIT DURCHMESSERSTEUERUNG
CATHÉTER À BALLONNET COMMANDÉ PAR DIAMÈTRE

(30) Priority: 21.12.2007 DK 200701849
(43) Date of publication of application: 08.09.2010
(73) Proprietor: Coloplast A/S, 3050 Humlebæk (DK)
(72) Inventor: SCHERTIGER, Lars Olav, DK-3480 Fredensborg (DK)
(86) International application number: PCT/DK2008/050330
(87) International publication number: WO 2009/080050

(56) References cited:
- US-A- 3 065 750
- US-A- 5 074 842
- US-A1- 2005 288 684

## Description

The invention relates to a diameter-controlled balloon catheter or probe. The catheter is to be used in connection with anal irrigation. The invention also relates to a method for performing anal irrigation.

### Background

Anal irrigation is one of a number of treatments used to aid people with bowel problems. People suffering from bowel problems are often paralyzed, typically due to spinal cord injuries, and confined to a wheelchair or hospitalized. In these situations, often the peristaltic functions, i.e. the reflexes and muscles of the bowel, cannot be stimulated correctly. This results in constipation or random discharge of bowel contents. By using anal irrigation a stimulation of the peristaltic movements of the colon can be provided.

To perform such anal irrigation, a device comprising an anal probe, also called anal catheter, rectal catheter or speculum, is provided. The anal probe is inserted into the rectum through the anus. The anal probe is typically retained in the rectum by retention means, most commonly a balloon, which is inflated against the wall of the rectum. A liquid, such as water or a saline solution, is then introduced into the rectum through the anal probe. The amount of liquid is generally up to 1.5 liters, depending on the person.

The anal probe is then removed followed by a discharge from the colon through the rectum. In some cases during anal irrigation the expansion of the rectum and the sphincter or the early stimulation of the peristaltic movements of the rectum may result in that the inflated balloon, or other fixating means, does not provide proper sealing and discharge of the rectum will thus occur around the anal probe. This undesired discharge can be very unpleasant for the patient as well as for the caring personnel performing the anal irrigation.

### Description of related art

US3065750 shows a sanitary douche which may be dilated upon passage therethrough of the fluid employed therewith. More specifically, it deals with a cylindrical unit surrounded by plastic bags or balloons which become dilated upon passage therethrough of sanitizing liquid under pressure.

US5074842 provides a system for anal irrigation which comprises a tube and a balloon for retaining the tube in the rectum. The balloon is a specially shaped balloon designed to retain the tube in the rectum and it can be inflated after the tube is inserted. Air to the balloon is delivered through a syringe valve into a line which communicates with an air passage. This configuration provides an integral double-lumen tube having one lumen or channel which allows the colon sweeping solution to enter the patient and a second channel which delivers or removes air from the balloon used to retain the tube in place

### Summary of the Invention

The invention concerns an anal probe for use in connection with anal irrigation, where the probe is provided with a balloon for retaining the probe in the rectum and where the balloon is inflated under influence of irrigation liquid. At some point during inflation, the balloon-element will be distended to such an extent that the balloon-element will contact the inner walls of the rectum. At this point, the balloon-element will begin to extend in the longitudinal direction, thereby opening a water channel to the rectum.

The probe may be provided with a proximal part, including a tip part, and a distal part, including a shaft part. A part of the proximal part is separate from the distal part so that this part can move longitudinally with respect to the distal part. The tip part and the shaft part each have a water channel and, in a first configuration of the probe, the water channels are not in communication with each other, while in a second configuration of the probe, the water channels are in contact with each other. The movable parts are biased together towards the first configuration by the balloon-element, providing a resilient element.

### Detailed Description of the Invention

In the first aspect, the invention relates to a probe for irrigating liquid into the human body, which probe comprises
- a tip part having a tip channel with an inlet and an outlet
- a shaft part having a shaft channel with an inlet, an outlet and a connector adapted for connection to irrigating liquid supply means
- a balloon for retaining the probe in the human body, which balloon is attached to encompass the outlet of the shaft channel and the inlet to the tip channel such that the balloon defines a lumen there between
where the outlet of the shaft channel is in fluid communication with the lumen of the balloon and where the probe is movable between
- a first configuration in which the inlet to the tip channel is not in fluid communication with the lumen of the balloon, and
- a second configuration in which the inlet to the tip channel is in fluid communication with the lumen of the balloon.

Such a probe is particularly easy to use, as a user only needs to attach a fluid supply including a pump to the probe and pump the irrigation liquid into the probe. As there is no communication between the water channels in the first configuration, the irrigation liquid will flow through the shaft channel and into the balloon lumen, thus inflating the balloon in this configuration. In the second configuration, there is communication between the shaft water channel and the tip water channel. In this configuration, the irrigation liquid will flow through the shaft channel into the balloon lumen and from there into the tip channel and further on into the opening to be irrigated. In other words, in a first configuration of the probe, the inlet of the tip channel is closed and, in a second configuration of the probe, the inlet of the tip channel is opened.

When such a probe is to be used, only one pumping action is needed as the same action will both activate the balloon and pump irrigation fluid into the body opening. Thus the user inserts the probe into the opening to be irrigated (rectum, vagina, stoma, urethra) and then starts pumping. The user only needs to continue pumping and does not need to switch from pumping the balloon to pumping irrigation liquid into the body opening - this is all controlled by the movement of the probe. The dilation of the balloon controls when the probe shifts from the first configuration to the second configuration..

The longitudinal part of the probe may be in the form of a rectal catheter having a coating so as to make the insertion easier. Typically the catheter will be a cylindrical body having a size of about 8-16 mm's as the diameter such as 10 mm's and a length of about 70-200 mm's such as about 150 mm's.

The probe may also be in the form of a urethral catheter having a size of about 8-18 French size, which corresponds to about 2,7 - 6,0 mm's in diameter. The insertable length would typically be about 250-300 for a male catheter and for a female catheter the insertable length would typically be between 60-130 mm. The insertable length corresponds to the length of the tubular element constituting the catheter except for the 3-5 cm's closest to the connector.

The probe may further be used for irrigation into a stoma, in which case a rectal catheter is used, and for vaginal irrigation.

The probe comprises a proximal part including a tip part and a distal part including a shaft part. The tip part includes a generally cylindrical element having a rounded end with eyelets defining the outlet of the probe. The shaft part also includes a generally cylindrical element with a connector at the distal end, defining the inlet to the probe. In this context and through-out the application, when referring to the proximal part or end, referral is made to the part or end nearest the insertion end. Correspondingly, when referring to the distal part or end, referral is made to the end opposite the insertion end - i.e. the connector end.

The tip part and the shaft part may be made of two separate parts which are joined at least in the first configuration. Alternatively, they may be made as one integral part. The tip part has a through-going water channel (a tip channel) having an inlet in or near the distal end and an outlet in the proximal end. The eyelets communicate with the water channel of the tip part so that an outlet of the water channel corresponds to an eyelet of the tip. The shaft part also includes a water channel (a shaft channel) having an inlet in connection with the connector and an outlet near the proximal end. The connector may be attached to the irrigation liquid supply.

The tip part may be provided with a cap situated at the outmost tip portion. The cap would to a certain degree cover the eyelets during insertion thereby preventing the eyelets from being blocked by faeces present in the rectum or colon (for anal and stomal irrigation, respectively). Furthermore, the tip may be provided with several eyelets, such as up to 10 or 15 or even more - thereby reducing the risk that all of them are blocked at the same time.

In an embodiment of the invention, the invention concerns a probe wherein a transition between the first configuration and the second configuration is controlled by the dilation of the balloon. This means that when the balloon has dilated transversely to such an extent that dilation is no longer possible in this direction, the balloon will dilate in the longitudinal direction, thereby initiating the transition between the first configuration and the second configuration.

In one embodiment of the invention, the tip part and the shaft part are elongated elements defining a longitudinal direction, a part of a proximal part of the probe is displaceable longitudinally with respect to a distal part of the probe and the balloon is attached to the distal part and the displaceable part of the probe.

By longitudinally displacement is meant a displacement generally in the longitudinal direction. In this embodiment, the balloon-element can displace a part of the proximal part of the probe with respect to the distal part when the balloon can no longer dilate in the transverse direction. The balloon-element is annularly attached to the distal part of the probe at a first attachment zone and further annularly attached to the displaceable proximal part of the probe at a second attachment zone. Thereby, the movement of the balloon-element will be transferred to the distal part and the displaceable proximal part. The shaft channel has an outlet in communication with the lumen of the balloon. In a first configuration of the probe, there is no communication between the two water channels of the probe, as the inlet to the tip channel is out of communication with the lumen. In the second configuration of the probe, the irrigation fluid may access the inlet of the tip channel and hence reach the outlet of this channel (in connection with the eyelets) and flow further into the body opening, e.g. the rectum or the urethra. The opening of the inlet to the tip channel is effected by the longitudinal dilation of the balloon-element, which dilation displaces the displaceable part of the probe.

A related embodiment relates to the tip part and the shaft part being separate elements with the balloon attached to the shaft part and the tip part and the tip part being displaced longitudinally with respect to the shaft part in the second configuration of the probe. In this embodiment, the displaceable part of the proximal part of the probe is the entire tip part.

Another embodiment relates to where the probe further comprises a sleeve extending longitudinally along at least a part of the tip part, where the balloon is attached to the shaft part and the sleeve and where the sleeve is displaced longitudinally with respect to the shaft part in the second configuration of the probe. In this embodiment, the sleeve constitutes the displaceable part of the proximal part of the probe. Thus, the tip part and the shaft part may be made as one integral element having two separate water channels. The sleeve will actually be displaced longitudinally with respect to the remaining parts of the probe.

In these embodiments, the balloon will expand predominantly in the transverse direction until the outer contours of the balloon gets into contact with the intestinal walls. At this point, the balloon will begin expanding predominantly in the longitudinal direction and thus pulling the movable parts from each other in said longitudinal direction. When the movable parts have separated a certain distance from each other, access to the tip channel will be provided and water will flow through the tip channel and further into the body opening to be irrigated. In this context, transverse and longitudinal directions are defined as the directions of the probe so that the longitudinal direction corresponds to the lengthwise direction of the probe and the transverse direction is transverse thereto. The probe is a generally cylindrical element having an inlet in one end and an outlet in the other, both generally along the axis of the cylindrical element. This axis defines the lengthwise direction.

If the tip part and the shaft part are two separate parts, the first configuration may correspond to a contracted position and the second configuration may correspond to an extended position along the longitudinal direction. This means that the tip part and the shaft part has a distance between them in the extended position and are in contact with each other in the contracted position. Then, the tip part and the shaft part may define contact surfaces in contact with each other in the first configuration of the probe. These contact surfaces may be provided as polished surfaces so that they define close and intimate contact to provide for a sealing between the surfaces. Such a sealing between smooth surfaces may be sufficient to ensure that, in this first configuration, most of the irrigation fluid is prevented from flowing through the tip part. In a related embodiment, the contact surfaces may be provided with an additional sealing element in form of a gasket.

When the tip part and the shaft part are separate parts, the inlet to the tip part may be placed in the contact surface of the tip part. This provides for a particularly simple solution, as the tip part may then be formed as a simple cylindrical element having a rounded tip. In another embodiment, either the tip part or the shaft part has a protrusion and the other part has an indentation corresponding to the protrusion. In such a solution, the inlet to the water channel of the tip part is hidden in either the indentation or at the protrusion, depending on whether the tip part has an indentation or a protrusion. Such solutions increase the ability of the probe to keep the inlet to the tip part closed in the first configuration. If the tip part has a protrusion and the shaft part has a corresponding indentation, then the inlet may be provided in the sidewall of the protrusion. In the first configuration, this inlet is hidden and closed due to the contact surfaces and the contact between the sidewalls of the protrusion and the sidewalls of the indentation in the shaft part. If the tip part has an indentation and the shaft part has a corresponding protrusion, the inlet may be provided anywhere in the indentation and it may be accessed or opened when the protrusion is completely removed from the indentation. In a related embodiment, the protrusion of the shaft portion may be provided with an additional water channel having an inlet in a sidewall of the protrusion and an outlet in the end of the protrusion. In this embodiment, the protrusion need not be completely removed from the indentation in order to create access to the inlet of the tip part.

If the probe comprises a sleeve as a part of the proximal part of the probe, this sleeve and the tip part should be sealed with respect to each other so that irrigation liquid is prevented from leaving the balloon-lumen. This sealing may be made by a gasket well-known in the art. The sleeve should be made of a material corresponding to the rest of the probe. In this embodiment, the inlet to the water channel of the tip may be placed below the sleeve in the first configuration of the probe. Thereby, longitudinal displacement of the sleeve will open the inlet to the tip water channel so that irrigation fluid will be able to flow through the probe into the rectum or the urethra. The sleeve-construction may be advantageous if the opening (e.g. rectum) is blocked by a blockage of faeces. In this situation, when the probe is inserted, it may be difficult for the tip to move forward due to the blockage. It may then be easier for the sleeve to move forward and in this way opening for communication for the irrigation liquid through the tip-channel.

The probe is provided with a balloon comprising a balloon element. The material of the balloon element is resilient under the normal use conditions - e.g. when subjected to pressures below 2.5 atm. The balloon element may be made of PU (Poly-Urethane), latex or PVC (Poly-vinyl-chloride), although other materials having similar properties may be used. Other types of material may be silicone elastomers (e.g. LSR, HTV, RTV, Addition Cure or Condensation Cure), natural rubber latex, isoprene rubber, chloroprene rubber (e.g. Neoprene ®), PU (poly-urethane, e.g. TPU or cross-linked), styrenic elastomers (TPE-S, e.g. Kraton ®, Septon ®, Sibstar ® reactor materials or compounds) and poly-ether block amide (TPE-A), e.g. Pebax ®).

The stretchability of the balloon element is such that it requires a strength of between about 0.1 MPa and about 10.0 MPa to extend the balloon to twice its length - preferably between about 0.2 MPa and about 1.5 MPa. In other words, when the strain has reached 100% of the initial length, the stress in the material is between about 0.1 MPa and about 10.0 MPa - preferably between about 0.2 MPa and about 1.5 MPa.

The balloon element is made of a material having a Shore-hardness of about 1-70 Shore A, preferably between about 10 and about 30 Shore A.

The balloon is in the form of a lumen-defining element (a balloon-element) attached to the distal part (the shaft part) below the outlet from the shaft water channel and the proximal part of the probe (either the tip part itself or the surrounding sleeve) above the inlet to the tip water channel. The balloon-element is preferably annularly attached to the shaft part and the proximal part, respectively. The attachment should be liquid-tight in the normal use conditions, e.g. when subjected to pressures below 2.5 atm. The attachment is preferably done by gluing.

In this embodiment, the user pumps irrigation liquid into the probe and the balloon-element will expand initially in the transverse direction and subsequently in the longitudinal direction. While the balloon-element is expanding (or dilating) in the transverse direction, it will also dilate at least slightly in the longitudinal direction. However, initially, the dilation will predominantly occur in the transverse direction until the outer contours of the balloon-element reaches the intestinal wall of the rectum or the internal wall of the urethra. At this point, the balloon is prevented from expanding further in the transverse direction and it will then predominantly dilate in the longitudinal direction. Since the balloon is in one end attached to the tip part and in the opposite end attached to the shaft part, the longitudinal dilation will pull the two parts away from each other along the longitudinal direction. When the balloon-element has dilated to a certain extent in the longitudinal direction, the two parts will be separated to such an extent that the water channel of the tip part will be in fluid communication with the lumen of the balloon, and in this position, irrigation fluid will be able to flow through the tip part and into the rectum or the urethra of the user. In other words, the tip channel will be opened as the balloon element has expanded to a certain extent in the longitudinal direction. At this point, the balloon will not expand any further but the continuous pumping will pump irrigation liquid directly through the probe and into the rectum or urethra.

During use of the irrigation system, particularly when used for anal irrigation, the intestinal wall may be subjected to peristaltic movement and then the intestinal wall may move outwards, thereby enlarging the circumference of the wall. The probe of the present invention will be able to compensate for this, as the balloon-element in this case will expand a little further in the transverse direction until the balloon-element has again reached contact with the intestinal wall. During this pulsating movement, the water flow through the probe may be disrupted but this will not affect the irrigation procedure negatively.

The probe is easy to remove after the irrigation procedure. If the balloon is not fully inflated, the probe may just be pulled out without resistance. If the balloon is fully inflated, pulling at the connector end will separate the tip part from the shaft part or separate the shaft part from the sleeve. Thereby, the inlet to the tip channel will be opened and the liquid stored in the balloon will flow into the irrigation opening (rectum, vagina, stoma or urethra). When the balloon is empty, the probe may easily be removed.

The irrigating liquid may be any suitable medium such as tap water, isotonic salt water, sterile water or oily substances.

In a second aspect, the invention relates to a method of performing anal irrigation using a probe comprising a distal part, a proximal part and a balloon, which is attached to the distal part and a displaceable part of the proximal part and defines a lumen therebetween, where the distal part comprises a shaft part made as a generally elongated element and the proximal part comprises a tip part made as a generally elongated element, which elongated elements define a longitudinal direction, which method comprises the steps of
- introducing the probe into the rectum,
- supplying irrigation fluid into the probe through a water channel of the shaft part and into the lumen by a first pumping action
- expanding the balloon by the same first pumping action
- moving the displaceable part of the proximal part longitudinally thereby providing access to an inlet of a water channel in the tip part by the same first pumping action
- supplying irrigation fluid through the tip part and into the rectum or urethra by the same first pumping action.

This method implies that the user introduces the probe, supplying irrigation fluid to the probe by a pumping action. Then the pumping action will provide for the other actions in succession. This makes this method very easy to use compared to known methods.
The connection between the probe and the irrigation fluid supply may be by means well known in the art. A simple solution will be to use a connector at an end of the shaft part and a tube connected to a closed bag. Then an air pump may be used to pump air into the bag and thereby eventually displacing the irrigation fluid from the bag. Other solutions are also possible.

In a third aspect, the invention relates to a method of performing anal irrigation using a probe comprising a distal part, a proximal part and a balloon, which is attached to the distal part and a displaceable part of the proximal part and defines a lumen therebetween, where the distal part comprises a shaft part made as a generally elongated element and the proximal part comprises a tip part made as a generally elongated element, which elongated elements define a longitudinal direction, which method comprises the steps of
- introducing the probe into the rectum,
- supplying irrigation fluid into the probe through a water channel of the shaft part and into the lumen
where the irrigation fluid will cause the balloon to expand transversely and subsequently longitudinally and thereby cause the probe to move between a first configuration in which the inlet to the tip channel is not in fluid communication with the lumen, and a second configuration in which the inlet to the tip channel is in fluid communication with the lumen.

Such a method is likewise easy to use as the user only connects the irrigation fluid supply to the probe and starts pumping the irrigation fluid. Then the probe will expand by itself until retention and then open for the water channel in the tip part. Thus, the user may use the same pumping action for pumping the retention means as well as the irrigation fluid. This method further ensures that the outer contours of the retention means will be in contact with intestinal walls at all times. Thus, the retention means will also be able to function as a sealing element in the intestine.

### Brief Description of the Drawing

Figures 1a and 1b illustrate an embodiment of the invention where the displaceable part of the proximal part is the tip part so that this tip part is displaceable with respect to the shaft part;
Figure 2 illustrates a related embodiment having another configuration of the joint between the tip part and the shaft part; and
Figures 3a and 3b illustrate an embodiment of the invention where the displaceable part of the proximal part is a sleeve, which is displaceable with respect to the shaft part.

### Detailed Description of the Drawing

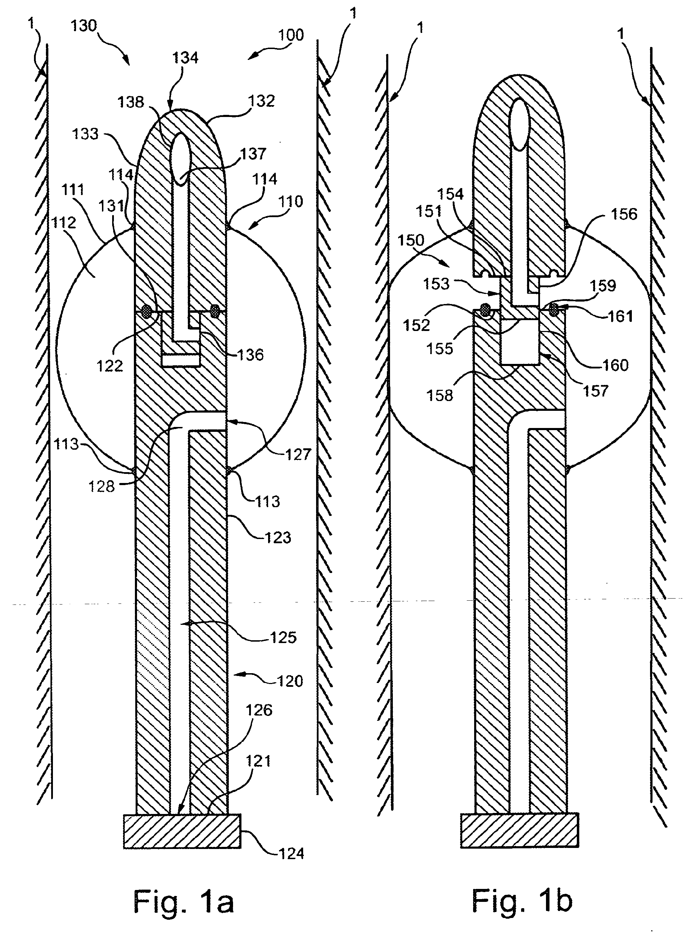

Figures 1a and 1b illustrate an embodiment of the invention where the displaceable part of the probe **100** corresponds to the tip part **130.** Figure 1a illustrates the probe **100** in a first configuration and figure 1b illustrates the same probe **100** in a second configuration. The probe **100** comprises a balloon **110** surrounding a joining part **150** of the probe. The joining part **150** defines the joint between the shaft part **120** and the tip part **130** of the probe. The shaft part **120** is defined as the part connected to the water supply and the tip part **130** is the other part. When defining the proximal end of the probe as the insertion end and the distal end as the other end, in this embodiment, the tip part **130** corresponds to the proximal part of the probe and the shaft part **120** corresponds to the distal part of **the** probe. In the illustrated embodiment, the balloon **110** comprises a balloon-element **111** defining a balloon lumen **112** between the inside of the balloon-element, the tip part and the shaft part. The balloon element **111** is provided as a cylindrical tubular sleeve and partly enclosing the tip part and the shaft part, and is in one end attached to the shaft part **120** by a first annular attachment **113** and likewise in the other end attached to the tip part **130** by a second annular attachment **114.** The annular attachments **113, 114** may be in the form of an adhesive The shaft part **120** has a distal end **121** and a proximal end **122** and a curved surface **123** extending there between so that the ends **121, 122** and the surface **123** combined make out a generally cylindrical element. The shaft part **120** also comprises a connector **124** in the distal end and a water channel **125** with a water inlet **126** and a balloon outlet **127.** In the embodiment illustrated in figures **1a** and 1b the water channel **125** comprises a bend **128,** which is illustrated as a 90 degrees bend. However, other forms of bends may also be used - e.g. a 45-degree bend. For practical purposes, at least a slight curve on the water channel **125** is preferred, as the balloon outlet **127** needs to communicate with the balloon lumen **112.** The tip part **130** comprises a distal end **131** and a proximal end **132** and a curved surface **133** extending there between. Like with the shaft part **120** these three parts make out a generally cylindrical element. The proximal end **132** corresponds to the actually tip **134** of the probe. The tip part **130** further comprises a water channel **135** having a balloon inlet **136** and a tip outlet **137.** The tip outlet **137** is provided in connection with eyelets **138** at the tip **134** of the probe. The joining part **150** comprises a ring surface **151** at the distal end **131** of the tip part **130** and a corresponding ring surface **152** at the proximal end **121** of the shaft part **120.** The two ring surfaces **151, 152** are closely attached in a contracted position of the probe - corresponding to the first configuration of the probe, while they are out of touch in an extended position - corresponding to the second configuration - of the probe. The joining part **150** further comprises a protrusion **153** extending from the tip part **130,** and which protrusion has a proximal end 154 and a distal end 155 and sidewalls 156 extending there between. The protrusion 153 is adapted to fit in an indentation 157 provided in the proximal end of the shaft part 120. The indentation 157 has a distal end 158 and a proximal end 159 and sidewalls 160 extending there between. In this embodiment, the balloon inlet 136 to the water channel 135 in the tip part 130 is provided in one of the sidewalls 156 of the protrusion. As illustrated, one of the contact surfaces 151, 152 may be provided with a sealing 161 so as to enhance the sealing effect between the two surfaces, when the probe is in the contracted position. The protrusion serves several purposes - one purpose is to provide a sealed inlet 136 to the water channel 135 of the tip part, another purpose is to provide a guide for the two parts (the tip part 130 and the shaft part **120**) of the probe. The sealed inlet may be provided as an inlet at the distal end 131 of the tip part **130**, particularly if the contact surfaces are provided with a sealing. Likewise, the guide may be provided by any other guide means extending between the two parts or it may be entirely omitted. Hence, the protrusion and indentation need not be there.

When the probe is to be used, the probe with the balloon in the collapsed position is inserted into the rectum. Water supply means is attached to the connector **124** at the distal end of the shaft part **120** of the probe **100.** Then irrigation fluid is injected or pumped by any traditional means into the water channel **125** and flows from there through the balloon outlet **127** into the balloon lumen **112** which is inflated by this. Initially, the balloon lumen is expanding in the radial direction (transverse to the longitudinal direction) of the probe, thereby actually enhancing the sealing between the tip part and the shaft part. This is illustrated in figure 1a, where the balloon element is not touching the internal wall 1 of the opening to be irrigated - e.g. the intestinal walls. When the balloon lumen reaches the internal wall it can no longer expand in the transverse direction but will begin expanding in the longitudinal direction of the probe, thereby extending the probe as the balloon is attached to the two parts. This is illustrated in figure 1b, where the balloon element **111** is in close contact with the internal wall 1. When the probe is extended to such an amount that the balloon inlet **136** to the water channel **135** of the tip part **130** is exposed, water will begin to flow through this water channel **135**, through the eyelets **138** and further on into the intestines, urethra or vagina. Should the intestinal walls dilate, the balloon will compensate by again expanding in the radial direction, thereby temporarily closing the inlet to the water channel. When the balloon **111** again reaches the intestinal wall, the balloon will expand in the longitudinal direction and again open the inlet to the water channel. This process may repeat itself several times during the anal irrigation.

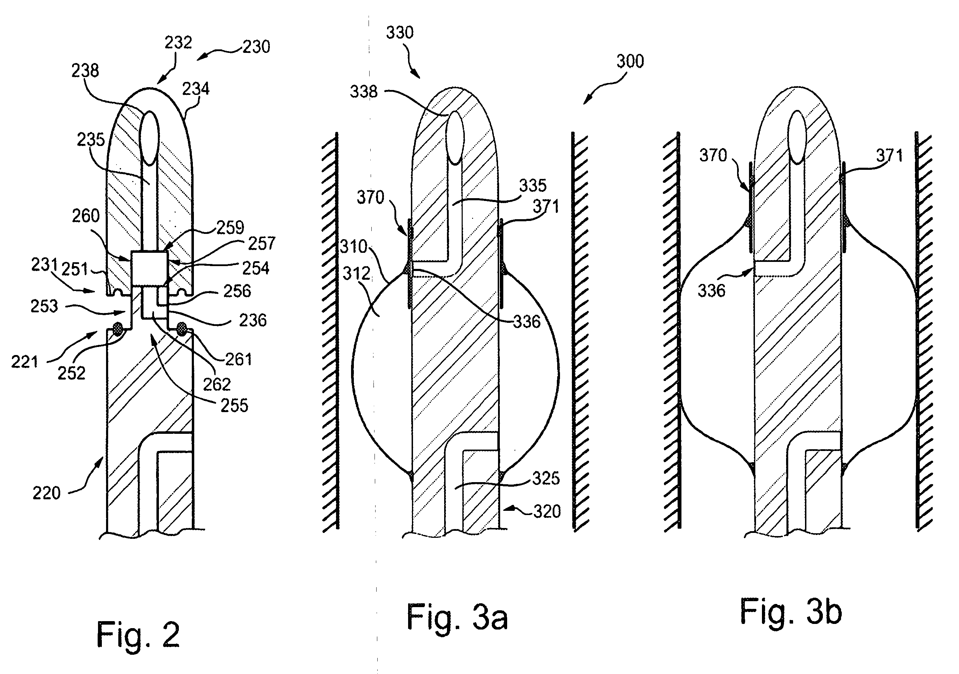

Figure 2 illustrates a probe 200 according to the invention similar to the one in figures 1a and 1b. One difference is the joint between the tip part and shaft part, which is made differently in this embodiment. Only parts of the probe are illustrated, the parts are numbered with numbers corresponding to the numbers used in figures 1a, 1b, except for the prefix being 2 instead of 1. The tip part 230 of figure 2 also comprises a proximal end 232 corresponding to the actual tip 234 and a distal end 231 and a curved surface 233 extending there between. Likewise, the shaft part 220 of the embodiment comprises a proximal end 222 and a distal end including the connector (not shown).The distal end 231 of the tip part forming part of the joint 250 comprises a ring surface 251. A corresponding ring surface 252 is provided at the proximal end 221 of the shaft part 220. Like the embodiment of figures 1 a and 1b, the joining part **250** further comprises a protrusion **253** and an indentation **257**. However, in figure 2, the protrusion **253** is extending from the shaft part **220** and the indentation **257** is provided in the distal end of the tip part **231**. The protrusion has a proximal end **254** and a distal end **255** and sidewalls **256** extending there between. In this embodiment, the protrusion **253** furthermore comprises a water channel **262** extending from the sidewalls **256** and to the proximal end **254**. The indentation is provided with the inlet **236** to the tip water channel **235**. When the tip part **230** is displaced longitudinally with respect to the shaft part **220**, the irrigation liquid in the balloon lumen (not shown) will be able to enter into the water channel **262** of the protrusion **253.** From there, it will flow through the inlet **236,** through the tip water channel **235** further through the eyelet(s) **238** and into the opening to be irrigated - e.g. the rectum, vagina, urethra or colon.

Figures 3a and 3b illustrate another probe according to the invention, where the displaceable part of the probe is a sleeve **370** disposed at the outer side of the tip part **330.** Again, corresponding reference numbers are used, except for the prefix now being 3. Figure 3a shows the embodiment in the first configuration and figure 3b shows the embodiment in the second configuration. In this embodiment, the tip part **330** and the shaft part **320** is one integrated element. However, the two parts **330**, **320** still have separate water channels, a shaft water channel **325** and a tip water channel **335.** The sleeve **370** will be in intimate contact with the outer side of the tip part **330** through a sealing element **371** disposed annularly around the tip part **330** between the tip part **330** and the sleeve **370**. Initially, the inlet **336** to the tip water channel **335** will be covered by the sleeve **370**. When the balloon **310** has dilated transversely, it will begin dilating longitudinally, thereby pulling the sleeve **370** in the proximal direction. When the sleeve **370** has been pulled a certain distance, the inlet **336** to the tip water channel **335** will be opened and in communication with the balloon lumen **312**. Thereby, the irrigation liquid will be able to flow through the tip water channel **335** and out through the eyelets **338** into the opening to be irrigated.

## Claims

1. A probe (100, 200, 300) for irrigating liquid into the human body, which probe (100, 200, 300) comprises
- a tip part (130, 230, 330) having a tip channel (135, 235, 335) with an inlet (136, 236, 336) and an outlet (137)
- a shaft part (120, 220, 320) having a shaft channel (125, 325) with an inlet (126), an outlet (127) and a connector (124) adapted for connection to irrigating liquid supply means
- a balloon (110) for retaining the probe in the human body, **characterized in that** the balloon (110) is attached to encompass the outlet (127) of the shaft channel and the inlet (136, 236, 336) to the tip channel such that the balloon (110) defines a lumen (112, 312) there between
where the outlet (127) of the shaft channel is in fluid communication with the lumen (112, 312) of the balloon and where the probe (100, 200, 300) is movable between
- a first configuration in which the inlet (136, 236, 336) to the tip channel is not in fluid communication with the lumen (112, 312) of the balloon, and
- a second configuration in which the inlet (136, 236, 336) to the tip channel is in fluid communication with the lumen (112, 312) of the balloon.

2. A probe (100, 200, 300) according to claim 1 wherein a transition between the first configuration and the second configuration is controlled by the dilation of the balloon (110).

3. A probe (100, 200, 300) according to claim 1 where the tip part (130, 230, 330) and the shaft part (120, 220, 320) are elongated elements defining a longitudinal direction, a part of a proximal part of the probe is displaceable longitudinally with respect to a distal part of the probe and where the balloon (110) is attached to the distal part and the displaceable part of the probe.

4. A probe (100, 200, 300) according to claim 2 where the tip part (130, 230, 330) and the shaft part (120, 220, 320) are separate elements with the balloon (110) attached to the shaft part (120, 220, 320) and the tip part (130, 230, 330) and where the tip part (130, 230, 330) is displaced longitudinally with respect to the shaft part (120, 220, 320) in the second configuration of the probe.

5. A probe (100, 200, 300) according to claim 4 where the tip part (130, 230, 330) and the shaft part (120, 220, 320) have contact surfaces (151, 152) in contact with each other in the first configuration of the probe.

6. A probe (100, 200, 300) according to claim 5 where the inlet (136, 236, 336) to the tip part is placed in the contact surface (151) of the tip part.

7. A probe (100, 200, 300) according to any of claims 4 or 5 where either the tip part (130, 230, 330) or the shaft part (120, 220, 320) has a protrusion (153) and the other part has an indentation (157) corresponding to the protrusion.

8. A probe (100, 200, 300) according to claim 7 where the inlet (136, 236, 336) to the tip part is placed in the protrusion (153).

9. A probe (100, 200, 300) according to claim 2 where the probe further comprises a sleeve (370) extending longitudinally along at least a part of the tip part (130, 230, 330), where the balloon (110) is attached to the shaft part (120, 220, 320) and the sleeve (370) and where the sleeve (370) is displaced longitudinally with respect to the shaft part (120, 220, 320) in the second configuration of the probe.

10. A probe (100, 200, 300) according to claim 9 where the inlet (136, 236, 336) to the tip channel is situated below the sleeve (370) in the first configuration of the probe.

11. A probe (100, 200, 300) according to any of the preceding claims where the balloon (110) is made of a resilient material.

## Patentansprüche

1. Sonde (100, 200, 300) zum Spülen einer Flüssigkeit in den menschlichen Körper, wobei die Sonde (100, 200, 300) Folgendes umfasst:
- einen Spitzenteil (130, 230, 330) mit einem Spitzenkanal (135, 235, 335) mit einem Einlass (136, 236, 336) und einem Auslass (137)
- ein Schaftteil (120, 220, 320) mit einem Schaftkanal (125, 325) mit einem Einlass (126), einem Auslass (127) und einem Verbindungsglied (124) zur Verbindung mit einem Spülflüssigkeit-Zuführungsmittel
- einen Ballon (110) zum Halten einer Sonde im menschlichen Körper, **dadurch gekennzeichnet, dass** der Ballon (110) so befestigt ist, dass er den Auslass (127) des Schaftkanals und den Einlass (136, 236, 336) zum Spitzenkanal umgibt, so dass der Ballon (110) ein Lumen (112, 312) dazwischen definiert
wobei der Auslass (127) des Schaftkanals mit dem Lumen (112, 312) des Ballons in Flüssigkeitsverbindung steht und wobei die Sonde (100, 200, 300) bewegt werden kann zwischen
- einer ersten Konfiguration, in welcher der Einlass (136, 236, 336) zum Spitzenkanal nicht mit dem Lumen (112, 312) des Ballons in Flüssigkeitsverbindung steht, und
- einer zweiten Konfiguration, in welcher der Einlass (136, 236, 336) zum Spitzenkanal mit dem Lumen (112, 312) des Ballons in Flüssigkeitsverbindung steht.

2. Sonde (100, 200, 300) nach Anspruch 1, worin ein Übergang zwischen der ersten Konfiguration und der zweiten Konfiguration durch die Aufweitung des Ballons (110) kontrolliert wird.

3. Sonde (100, 200, 300) nach Anspruch 1, worin der Spitzenteil (130, 230, 330) und der Schaftteil (120, 220, 320) längliche Elemente sind, die eine Längsrichtung definieren, wobei ein Teil eines proximalen Teils der Sonde in Längsrichtung relativ zu einem distalen Teil der Sonde verschoben werden kann und worin der Ballon (110) am distalen Teil und am verschiebbaren Teil der Sonde befestigt ist.

4. Sonde (100, 200, 300) nach Anspruch 2, worin der Spitzenteil (130, 230, 330) und der Schaftteil (120, 220, 320) separate Elemente sind, wobei der Ballon (110) am Schaftteil (120, 220, 320) und am Spitzenteil (130, 230, 330) befestigt ist und wobei der Spitzenteil (130, 230, 330) in Längsrichtung relativ zum Schaftteil (120, 220, 320) in der zweiten Konfiguration der Sonde verschoben wird.

5. Sonde (100, 200, 300) nach Anspruch 4, worin der Spitzenteil (130, 230, 330) und der Schaftteil (120, 220, 320) Kontaktflächen (151, 152) aufweisen, die in der ersten Konfiguration der Sonde miteinander in Berührung stehen.

6. Sonde (100, 200, 300) nach Anspruch 5, worin der Einlass (136, 236, 336) zum Spitzenteil in der Kontaktfläche (151) des Spitzenteils angeordnet ist.

7. Sonde (100, 200, 300) nach einem der Ansprüche 4 oder 5, worin entweder der Spitzenteil (130, 230, 330) oder der Schaftteil (120, 220, 320) einen Vorsprung (153) aufweist und der andere Teil eine dem Vorsprung entsprechende Einbuchtung (157) aufweist.

8. Sonde (100, 200, 300) nach Anspruch 7, worin der Einlass (136, 236, 336) zum Spitzenteil im Vorsprung (153) angeordnet ist.

9. Sonde (100, 200, 300) nach Anspruch 2, worin die Sonde ferner eine Hülse (370) umfasst, die sich zumindest entlang eines Teils des Spitzenteils (130, 230, 330) in Längsrichtung erstreckt, worin der Ballon (110) am Schaftteil (120, 220, 320) und der Hülse (370) befestigt ist und worin die Hülse (370) in Längsrichtung relativ zum Schaftteil (120, 220, 320) in der zweiten Konfiguration der Sonde verschoben wird.

10. Sonde (100, 200, 300) nach Anspruch 9, worin der Einlass (136, 236, 336) zum Spitzenteil in der ersten Konfiguration der Sonde unter der Hülse (370) angeordnet ist.

11. Sonde (100, 200, 300) nach einem der vorhergehenden Ansprüche, worin der Ballon (110) aus einem nachgiebigen Material besteht.

## Revendications

1. Sonde (100, 200, 300) pour irriguer un corps humain avec un liquide, ladite sonde (100, 200, 300) comprenant:
- une partie de pointe (130, 230, 330) qui présente un canal de pointe (135, 235, 335) pourvu d'une entrée (136, 236, 336) et d'une sortie (137);
- une partie d'arbre (120, 220, 320) qui présente un canal d'arbre (125, 325) pourvu d'une entrée (126), d'une sortie (127) et d'un connecteur (124) adapté pour être connecté à des moyens d'alimentation de liquide d'irrigation; et
- un ballonnet (110) pour retenir la sonde dans le corps humain, **caractérisée en ce que** le ballonnet (110) est attaché de manière à envelopper la sortie (127) du canal d'arbre et l'entrée (136, 236, 336) vers le canal de pointe de telle sorte que le ballonnet (110) définisse une lumière (112, 312) entre celles-ci,
dans laquelle la sortie (127) du canal d'arbre est en communication fluidique avec la lumière (112, 312) du ballonnet, et dans laquelle la sonde (100, 200, 300) est mobile entre:
- une première configuration, dans laquelle l'entrée (136, 236, 336) vers le canal de pointe n'est pas en communication fluidique avec la lumière (112, 312) du ballonnet; et
- une deuxième configuration, dans laquelle l'entrée (136, 236, 336) vers le canal de pointe est en communication fluidique avec la lumière (112, 312) du ballonnet.

2. Sonde (100, 200, 300) selon la revendication 1, dans laquelle une transition entre la première configuration et la deuxième configuration est commandée par la dilatation du ballonnet (110).

3. Sonde (100, 200, 300) selon la revendication 1, dans laquelle la partie de pointe (130, 230, 330) et la partie d'arbre (120, 220, 320) sont des éléments allongés qui définissent une direction longitudinale, une partie d'une extrémité proximale de la sonde est déplaçable de façon longitudinale par rapport à une partie distale de la sonde, et dans laquelle le ballonnet (110) est attaché à la partie distale et à la partie déplaçable de la sonde.

4. Sonde (100, 200, 300) selon la revendication 2, dans laquelle la partie de pointe (130, 230, 330) et la partie d'arbre (120, 220, 320) sont des éléments séparés avec le ballonnet (110) qui est attaché à la partie d'arbre (120, 220, 320) et à la partie de pointe (130, 230, 330), et dans laquelle la partie de pointe (130, 230, 330) est déplacée de façon longitudinale par rapport à la partie d'arbre (120, 220, 320) dans la deuxième configuration de la sonde.

5. Sonde (100, 200, 300) selon la revendication 4, dans laquelle la partie de pointe (130, 230, 330) et la partie d'arbre (120, 220, 320) possèdent des surfaces de contact (151, 152) qui sont en contact l'une avec l'autre dans la première configuration de la sonde.

6. Sonde (100, 200, 300) selon la revendication 5, dans laquelle l'entrée (136, 236, 336) vers la partie de pointe est disposée dans la surface de contact (151) de la partie de pointe.

7. Sonde (100, 200, 300) selon l'une quelconque des revendications 4 ou 5, dans laquelle la partie de pointe (130, 230, 330) ou la partie d'arbre (120, 220, 320) comporte une saillie (153), et l'autre partie présente une indentation (157) qui correspond à la saillie.

8. Sonde (100, 200, 300) selon la revendication 7, dans laquelle l'entrée (136, 236, 336) vers la partie de pointe est disposée dans la saillie (153).

9. Sonde (100, 200, 300) selon la revendication 2, dans laquelle la sonde comprend en outre un manchon (370) qui s'étend de façon longitudinale le long d'au moins une partie de la partie de pointe (130, 230, 330), dans laquelle la ballonnet (110) est attaché à la partie d'arbre (120, 220, 320) et au manchon (370), et dans laquelle le manchon (370) est déplacé de façon longitudinale par rapport à la partie d'arbre (120, 220, 320) dans la deuxième configuration de la sonde.

10. Sonde (100, 200, 300) selon la revendication 9, dans laquelle l'entrée (136, 236, 336) vers le canal de pointe est située en dessous du manchon (370) dans la première configuration de la sonde.

11. Sonde (100, 200, 300) selon l'une quelconque des revendications précédentes, dans laquelle le ballonnet (110) est constitué d'un matériau élastique.
